(19) Europäisches Patentamt
European Patent Office
Office européen des brevets

(11) Numéro de publication: **0 298 870 B1**

(12) **FASCICULE DE BREVET EUROPEEN**

(45) Date de publication du fascicule du brevet: **16.01.91**

(51) Int. Cl.⁵: **C 07 C 19/08,** C 07 C 17/22

(21) Numéro de dépôt: **88401776.5**

(22) Date de dépôt: **07.07.88**

(54) **Synthèse des bromures de perfluoroalkyle.**

(30) Priorité: **10.07.87 FR 8709870**

(43) Date de publication de la demande: **11.01.89 Bulletin 89/02**

(45) Mention de la délivrance du brevet: **16.01.91 Bulletin 91/03**

(84) Etats contractants désignés: **AT BE CH DE ES FR GB GR IT LI LU NL SE**

(56) Documents cités: **FR-A-1 512 068**

(73) Titulaire: **ATOCHEM**
**4 & 8, Cours Michelet La Défense 10**
**F-92800 Puteaux (FR)**

(72) Inventeur: **Drivon, Gilles**
**3 Rue Joanny Courbière**
**F-69850 Saint-Martin en Haut (FR)**
Inventeur: **Durual, Pierre**
**Les Essarts no. 2 Chemin du Rossignol**
**F-69390 Vernaison (FR)**
Inventeur: **Gurtner, Bernard**
**5 Boulevard Agutte Sambat**
**F-38000 Grenoble (FR)**
Inventeur: **Lantz, André**
**Domaine de la Hêtraie**
**F-69390 Vernaison (FR)**

(74) Mandataire: **Leboulenger, Jean et al**
**ATOCHEM Département Propriété Industrielle**
**F-92091 Paris la Défense 10 Cédex 42 (FR)**

**EP 0 298 870 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne le domaine des hydrocarbures aliphatiques perhalogénés et a plus particulièrement pour objet la préparation des bromures de perfluoroalkyle ou bromoperfluoroalcanes $R_F$-Br, $R_F$ désignant un radical perfluoroalkyle $C_nF_{2n+1}$, linéaire ou ramifié, contenant de 1 à 20 atomes de carbone.

Ces composés connus sont utilisés dans de nombreux domaines, en particulier en médecine comme radiopaques (agents de contraste aux rayons X) ou comme transporteurs d'oxygène dans les substituts de sang. Un composé plus spécialement étudié dans ce domaine est le bromure de n.perfluorooctyle $C_8F_{17}Br$.

Parmi les méthodes connues pour préparer ces composés, on peut signaler plus particulièrement:

—l'action du brome sur un composé $R_f$-$SF_5$ à 500°C en présence de nickel (brevet US 3 456 024),

—la photolyse en phase gazeuse d'un mélange d'hydrogéno-1 perfluoroalcane et de BrCl ou BrF (Adcock et al., Chem. Abstr. 100:34092 e),

—l'action du brome sur les composés $R_F$-I en présence d'un initiateur radicalaire comme l'AIBN (demande japonaise Kokai 85-184033),

—la photobromation de ces mêmes composés iodés par irradiation aux UV (Huang et al., Huaxue Xuebao 1984, 42(10) 1106-8 résumé dans Chem. Abstr. 102:78312 x).

La faiblesse des rendements obtenus et/ou la cinétique lente de ces méthodes font qu'elles ne permettent pas la production économique des bromures de perfluoroalkyle à l'échelle industrielle. Vu l'importance de ces composés dans le domaine médical, il est du plus grand intérêt de pouvoir les fabriquer au plus bas coût possible.

Il a maintenant été trouvé une technique permettant de fabriquer en une seule étape, avec un excellent rendement et une très bonne sélectivité les bromures de perfluoroalkyle à partir des chlorures de perfluoroalcane-sulfonyle correspondants $R_F$-$SO_2Cl$. Par rapport aux méthodes existantes, le procédé selon l'invention correspondant au schéma réactionnel suivant:

$$R_F\text{-}SO_2Cl + HBr \xrightarrow{\text{cata}/\Delta} R_FBr + SO_2 + HCl$$

offre en outre de nombreux avantages, notamment:

—simplicité technologique: utilisation de matériels classiques (réacteur agité, température modérée, pression atmosphérique, etc. . .),

—cinétique rapide, d'où une productivité élevée pouvant atteindre environ 0,5 mole/heure/litre de réacteur,

—sécurité d'exploitation: élimination simultanée et contrôlée d'acide chlorydrique et d'anhydride sulfureux.

Le procédé selon l'invention est caractérisé en ce qu'il consiste à faire réagir le bromure d'hydrogène gazeux sur un chlorure de perfluoroalcanesulfonyle en présence d'un catalyseur constitué par une amine ou une phosphine tertiaire ou un sel d'ammonium ou de phosphonium quaternaire à une température pouvant aller de 80 à 200°C et, de préférence, comprise entre 90 et 150°C. La température optimale dépend du nombre d'atomes de carbone du sulfochlorure utilisé et de sa pureté. Ainsi, par exemple, pour le sulfochlorure $C_8F_{17}SO_2Cl$ fraîchement distillé, elle est d'environ 120—140°C et pour $C_6F_{13}SO_2Cl$ d'environ 90—120°C.

Le procédé selon l'invention est de préférence mis en oeuvre à la pression atmosphérique, mais on ne sortirait pas du cadre de l'invention en travaillant sous légère pression ou dépression. On peut opérer dans un réacteur muni de dispositifs classiques d'agitation, de chauffage et d'introduction de gaz et surmonté d'un réfrigérant relié à un dispositif d'abattage des évents (HCl, $SO_2$ et HBr non transformé).

La quantité de catalyseur à utiliser peut varier dans de larges limites, mais est généralement comprise entre 0,1 et 5 moles pour 100 moles de chlorure de perfluoroalcane-sulfonyle et, de préférence, d'environ 1 à 2 moles pour 100. Comme exemples de catalyseurs à utiliser selon l'invention, on peut citer la méthyldioctylamine, la triphénylphosphine et plus particulièrement le bromure de tétrabutylammonium, le chlorure de méthyl trioctyl ammonium, le chlorure de diméthyl dioctyl ammonium et le bromure de tétrabutylphosphonium.

Bien qu'on puisse opérer au sein d'un solvant inerte vis-à-vis du bromure d'hydrogène, on préfère opérer en l'absence de solvant en veillant à ce que la température choisie pour la réaction soit suffisante pour liquéfier le milieu réactionnel.

Le débit de bromure d'hydrogène gazeux que l'on introduit dans le mélange réactionnel constitué par le chlorure de perfluoroalcanesulfonyle de préférence fraîchement distillé, et le catalyseur et porté à la température convenable, peut varier dans le larges limites. Un débit rapide favorise la productivité tandis qu'un débit lent favorise le rendement par rapport au HBr engagé. Sans que cela puisse constituer une quelconque limitation au domaine de l'invention, un débit horaire de bromure d'hydrogène de l'ordre de 0,1 à 0,5 moles par mole de chlorure de perfluoroalcanesulfonyle s'est avéré particulièrement favorable. Quelque soit le débit choisi, la réaction est considérée comme terminée lorsqu'il n'y a plus de dégagement de $SO_2$ ni de HCl dans les évents.

Les exemples suivants illustrent l'invention sans la limiter.

### Exemple 1

Dans un réacteur de 250 ml, équipé d'un agitateur rotatif (~500 tr/min), d'une prise de température, d'un injecteur de gaz et d'un réfrigérant à eau froide relié à un barboteur, on place 282 g de chlorure de perfluoro-octanesulfonyle brut pureté: 92% soit 0,5 mole de $C_8F_{17}$-$SO_2Cl$) et 3,2 g (0,01 mole) de bromure de tétrabutylammonium.

On porte le mélange à environ 125°C et, sous agitation, on y introduit pendant 4 heures et

demie du bromure d'hydrogène gazeux à un débit de 0,13 mole/heure. Le dosage des évents gazeux indique un dégagement total de 0,445 mole de HCl, 0,11 mole de HBr et de 0,47 mole de $SO_2$.

On dégaze ensuite le milieu réactionnel à l'azote pendant une heure, puis on filtre à température ambiante pour éliminer le solide surnageant. On récupère ainsi 250,5 g d'un liquide rougebrique dont l'analyse CPG montre qu'il contient 0,47 moles de $C_8F_{17}Br$, soit un rendement de 94%.

Après élimination de l'acidité résiduelle et distillation à pression atmosphérique (Eb=146°C), on obtient un produit de pureté supérieure à 99,5%.

Exemple 2

On opère comme à l'exemple 1, mais en remplaçant le bromure de tétrabutylammonium par 3,4 g (0,01 mole) de bromure de tétrabutylphosphonium.

On obtient 250,2 g de produit brut à 90,7% de $C_8F_{17}Br$ qu'on peut purifier comme à l'exemple 1.

Exemple 3

On opère comme à l'exemple 1, mais en remplaçant le bromure de tétrabutylammonium par 4 g (0,01 mole) de chlorure de méthyl trioctyl ammonium (Aliquat 336) et en introduisant le bromure d'hydrogène à un débit de 0,17 mole/heure pendant 5 heures.

On obtient 254 g de produit brut contenant 0,43 mole de $C_8F_{17}Br$. Rendement: 86%.

Exemple 4

Dans un réacteur de 2 litres équipé d'une agitation mécanique, d'une prise de température, d'une canne d'injection de gaz et d'un réfrigérant ascendant relié à une colonne d'abattage à l'eau, on charage 1847 g de $C_8F_{17}SO_2Cl$ ayant une pureté CPG de 90,5% (soit 3,22 moles de $C_8F_{17}SO_2Cl$) et 22,4 g de bromure de tétrabutylammonium.

On porte le mélange réactionnel à environ 125°C et on introduit pendant 4 heures HBr anhydre à un débit de 1 mole/heure.

Après dégazage pendant 1 heure à l'azote et élimination par filtration à température ambient du solide surnageant, on récupère un liquide rouge-brique pesant 1730 g et ayant une pureté CPG de 88,5%, soit 3,07 moles de $C_8F_{17}Br$ ce qui correspond à un rendement de 95%.

Par distillation à pression atmosphérique de ce brut réactionnel, on recueille un produit ayant une pureté⩾99,5%.

Le dosage des évents gazeux indique un dégagement total au cours de la réaction de 3,09 moles d'HCl et de 2,90 moles de $SO_2$.

Exemple 5

On opère comme à l'exemple 1, mais en remplaçant le bromure de tétrabutylammonium par 2,5 g (0,01 mole) de méthyl dioctylamine.

On obtient 256,5 g de produit brut à 61,5% de $C_8F_{17}Br$. Rendement: 63%.

Exemple 6

Si on opère comme à l'exemple 1, mais en remplaçant le bromure de tétrabutylammonium par 2,6 g (0,01 mole) de triphénylphosphine, on récupère 278 g de produit brut à 57% de $C_8F_{17}Br$. Rendement: 64%.

Exemple 7

Dans le même appareillage qu'à l'exemple 1, on charge 251 g de chlorure de perfluorohexanesulfonyle fraîchement distillé (pureté 99,4%), soit 0,6 mole de $C_6F_{13}SO_2Cl$, et 3,9 g de bromure de tétrabutylammonium.

On porte le mélange réactionnel à 120°C et on introduit pendant 5 heures HBr anhydre à un débit compris entre 0,3 et 0,1 mole/heure. La température du milieu réactionnel se stabilise rapidement à 95—100°C à cause du reflux du $C_6F_{13}Br$ formé. Le dosage des évents gazeux indique un dégagement total de 0,58 mole de HCl, 0,575 mole de $SO_2$ et 0,68 mole de HBr.

Après dégazage à l'azote pendant une heure et décantation du catalyseur, on récupère 229 g d'une phase organique contenant 96,8% de $C_6F_{13}Br$, soit un rendement de 92,6%.

**Revendications**

1. Procédé de préparation des bromures de perfluoroalkyle, caractérisé en ce qu'il consiste à faire réagir le bromure d'hydrogène gazeux sur un chlorure de perfluoroalcane-sulfonyle en présence d'un catalyseur constitué parune amine ou une phosphine tertiaire ou un sel d'ammonium ou de phosphonium quaternaire à une température allant de 80 à 200°C.

2. Procédé selon la revendication 1, dans lequel on met en oeuvre de 0,1 à 5 moles de catalyseur pour 100 moles de chlorure de perfluoroalcane-sulfonyle, de préférence environ 1 à 2 moles pour 100.

3. Procédé selon la revendication 1 ou 2, dans lequel le catalyseur est le bromure de tétrabutylammonium, le chlorure de méthyl trioctyl ammonium, le chlorure de diméthyl dioctyl ammonium ou le bromure de tétrabutylphosphonium.

4. Procédé selon l'une des revendications 1 à 3, dans lequel on opère entre 90 et 150°C.

5. Procédé selon l'une des revendications 1 à 4, dans lequel le débit horaire de bromure d'hydrogène est de l'ordre de 0,1 à 0,5 mole par mole de chlorure de perfluoroalcane-sulfonyle.

6. Procédé selon l'une des revendications 1 à 5, dans lequel on part du chlorure de perfluorooctane-sulfonyle pour former le bromure de perfluorooctyle.

**Patentansprüche**

1. Verfahren zur Herstellung von Perfluoralkylbromiden, dadurch gekennzeichnet, daß man gasförmigen Bromwasserstoff mit einem Perfluoralkansulfonylchlorid bei einer Temperatur von 80 bis 200°C in Gegenwart eines Katalysators reagieren läßt, der aus einem tertiären Amin oder

Phosphin oder einem quartären Ammonium- oder Phosphoniumsalz zusammengesetzt ist.

2. Verfahren nach Anspruch 1, worin man 0,1 bis 5 mol Katalysator pro 100 mol und vorzugsweise etwa 1 bis 2 mol pro 100 mol Perfluoralkansulfonylchlorid einsetzt.

3. Verfahren nach Anspruch 1 oder 2, worin der Katalysator Tetrabutylammoniumbromid, Methyltrioctylammoniumchlorid, Dimethyldioctylammoniumchlorid oder Tetrabutylphosphoniumbromid ist.

4. Verfahren nach einem der Ansprüche 1 bis 3, worin man zwischen 90 und 150°C arbeitet.

5. Verfahren nach einem der Ansprüche 1 bis 4, worin der stündliche Durchsatz des Bromwasserstoffs in der Größenordnung von 0,1 bis 0,5 mol pro mol Perfluoralkansulfonylchlorid beträgt.

6. Verfahren nach einem der Ansprüche 1 bis 5, worin man vom Perfluoroctansulfonylchlorid zur Bildung des Perfluoroctylbromids ausgeht.

## Claims

1. Process for the preparation of perfluoroalkyl bromides, characterized in that it consists in reacting gaseous hydrogen bromide with a perfluoroalkanesulfonyl chloride in the presence of a catalyst consisting of a tertiary amine or phosphine or a quaternary ammonium or phosphonium salt at a temperature ranging from 80 to 200°C.

2. Process according to Claim 1, in which 0.1 to 5 moles of catalyst are used per 100 moles of perfluoroalkanesulfonyl chloride, preferably approximately 1 to 2 moles per 100.

3. Process according to Claim 1 or 2, in which the catalyst is tetrabutylammonium bromide, methyltrioctylammonium chloride, dimethyldioctylammonium chloride or tetrabutylphosphonium bromide.

4. Process according to one of Claims 1 to 3, in which the operation is carried out between 90 and 150°C.

5. Process according to one of Claims 1 to 4, in which the hourly flow rate of hydrogen bromide is of the order of 0.1 to 0.5 mole per mole of perfluoroalkanesulfonyl chloride.

6. Process according to one of Claims 1 to 5, in which perfluorooctyl bromide is formed by starting from perfluorooctanesulfonyl chloride.